(19) Europäisches Patentamt
European Patent Office
Office européen des brevets

(11) Veröffentlichungsnummer: **0 219 652 A2**

(12) **EUROPÄISCHE PATENTANMELDUNG**

(21) Anmeldenummer: **86111764.6**

(22) Anmeldetag: **25.08.86**

(51) Int. Cl.[4]: **C 07 C 47/293**
C 07 C 45/65

(30) Priorität: **24.10.85 DE 3537813**

(43) Veröffentlichungstag der Anmeldung: **29.04.87 Patentblatt 87/18**

(84) Benannte Vertragsstaaten: **AT BE CH DE FR GB IT LI NL SE**

(71) Anmelder: **Degussa Aktiengesellschaft
Weissfrauenstrasse 9
D-6000 Frankfurt am Main 1(DE)**

(72) Erfinder: **Andrade, Juan, Dr.
545 Barnett Place
Ridgewood New Jersey 07450(US)**

(72) Erfinder: **Prescher, Günter, Dr.
Liesingstrasse 2
D-6450 Hanau 9(DE)**

(72) Erfinder: **Köhler, Klaus
Kettelerstrasse 37
D-6450 Hainburg(DE)**

(54) **Verfahren zur Herstellung von 1-Methylcyclopropan-carboxaldehyd.**

(57) 1-Methylcyclopropancarboxaldehyd der Formel

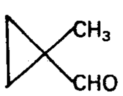

(I)

wird dadurch hergestellt, daß man 4-Chlor-2-methyl-butanal der Formel

$$ClH_2C - CH_2 - \underset{\displaystyle CH_3}{\underset{|}{CH}} - CHO \qquad (II)$$

bei einer Temperatur zwischen −40 und +70 °C in einem inerten Lösungsmittel mit der 1,0- bis 10,0-fachen molaren Menge an einer starken, nicht nucleophilen Base behandelt.

EP 0 219 652 A2

D e g u s s a Aktiengesellschaft
Weissfrauenstrasse 9, 6000 Frankfurt

# Verfahren zur Herstellung von 1-Methylcyclopropancarboxaldehyd

## Beschreibung:

Die Erfindung betrifft ein neues Verfahren zur Herstellung von 1-Methylcyclopropancarboxaldehyd der Formel

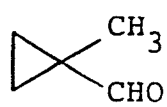

(I)

Dieses Cyclopropanderivat ist ein wichtiges Ausgangsmaterial für eine Synthese von Isodehydroabietenolid, welches seinerseits ein Zwischenprodukt für die Synthese des Antileukämie-Mittels Triptolide ist [vgl. J. Am. Chem. Soc. 101, 7423 (1979)].

Es ist bereits bekannt [vgl. J. Org. Chem. 27, 51 (1962)], 1-Methylcyclopropancarboxaldehyd durch Reduktion von 1-Methylcyclopropancarbonitril mit Lithiumaluminiumhydrid herzustellen. Das 1-Methylcyclopropancarbonitril seinerseits kann [vgl. J. Am. Chem. Soc. 56, 2710 (1934)] durch Umsetzung von Methacrylnitril mit Diazomethan hergestellt werden. Der Umgang mit Diazomethan ist jedoch für eine

Produktion in technischem Maßstab wenig geeignet.

Es ist auch bereits bekannt [vgl. J. Am. Chem. Soc. 97, 2778 (1975)], 1-Methylcyclopropancarboxaldehyd durch Umsetzung von Methallylalkohol mit Dijodmethan und trockener Zn-Cu-Legierung und anschließende Oxidation des dabei gebildeten 1-Methylcyclopropylcarbinols mit $CrO_3$ in Pyridin herzustellen. Die Gesamtausbeute an 1-Methylcyclopropancarboxaldehyd ist jedoch bei diesem Verfahren gering.

Das erfindungsgemäße Verfahren ist nun dadurch gekennzeichnet, daß man 4-Chlor-2-methyl-butanal der Formel

$$ClH_2C - CH_2 - \underset{\displaystyle CH_3}{\underset{|}{CH}} - CHO \qquad (II)$$

bei einer Temperatur zwischen -40 und +70 °C in einem inerten Lösungsmittel mit der 1,0- bis 10,0-fachen molaren Menge an einer starken, nicht nucleophilen Base behandelt.

Die Behandlung mit der Base wird vorzugsweise bei einer Temperatur zwischen -20 und +20 °C vorgenommen, die Base wird vorzugsweise in der 1,0- bis 4,0-fachen molaren Menge eingesetzt.

Das als Ausgangsmaterial dienende 4-Chlor-2-methyl-butanal der Formel (II) kann beispielsweise so hergestellt werden, daß man 1,1-Dimethoxy-2-methyl-4-hydroxy-butan bei einer Temperatur zwischen -20 und +80 °C in Gegenwart der 0,1- bis 20,0-fachen molaren Menge an Triphenylphosphin mit der 1,0- bis 10,0-fachen molaren Menge an Tetrachlorkohlenstoff umsetzt und das dabei gebildete 1,1-Dimethoxy-2-methyl-4-chlor-butan in saurem Medium hydrolysiert.

Geeignete inerte Lösungsmittel für das erfindungsgemäße Verfahren sind beispielsweise Ether, wie Diethylether, Diisopropylether oder Diethylenglykoldimethylether, und Kohlenwasserstoffe, wie Benzol oder Decalin.

Geeignete starke, nicht nucleophile Basen sind beispielsweise Natriumhydrid, tert.Butyllithium oder Lithiumdialkylamide, wie Lithiumdiisopropylamid.

Das erfindungsgemäße Verfahren kann beispielsweise so durchgeführt werden, daß man die Base in dem inerten Lösungsmittel suspendiert und das 4-Chlor-2-methyl-butanal unter Kühlung auf eine Temperatur zwischen -40 und +10 °C langsam zutropft. Danach kann das Reaktionsgemisch langsam auf Raumtemperatur gebracht und gegebenenfalls noch kurze Zeit auf eine höhere Temperatur, beispielsweise zwischen 30 und 70 °C,erwärmt werden.

Die Aufarbeitung des rohen Reaktionsgemisches kann beispielsweise so erfolgen, daß man unter Kühlung langsam Wasser zutropft, bis die Gasentwicklung beendet ist, und dann die Wasserphase abtrennt und verwirft. Die organische Phase wird neutral gewaschen, dann, beispielsweise über $MgSO_4$, getrocknet und schließlich fraktioniert destilliert. Gegebenenfalls kann auch auf das Abtrennen, Auswaschen und Trocknen der organischen Phase verzichtet und nach beendeter Wasserzugabe und Gasentwicklung direkt fraktioniert destilliert werden. Dabei tritt jedoch häufig eine Ausbeuteverminderung ein, was diese Arbeitsweise weniger empfehlenswert macht.

Die Erfindung wird durch die nachfolgenden Beispiele näher erläutert:

Beispiel 1:

78,3 g (0,65 Mol) 4-Chlor-2-methyl-butanal wurden bei +5 °C zu einer Suspension von 18,7 g (0,78 Mol) Natriumhydrid in 500 ml absolutem Diethylether langsam innerhalb von 30 Minuten zugetropft. Nach beendeter Zugabe wurde das Reaktionsgemisch auf Raumtemperatur kommen gelassen und danach noch 2 Stunden auf Rückflußtemperatur erwärmt. Anschließend wurde unter Kühlung langsam Wasser zugetropft, wodurch eine exotherme Reaktion unter Gasentwicklung einsetzte. Nach beendeter Gasentwicklung wurde die wäßrige Phase abgetrennt und verworfen. Die organische Phase wurde mehrmals mit Wasser ausgewaschen und dann über $MgSO_4$ getrocknet. Das $MgSO_4$ wurde abfiltriert, die organische Phase wurde unter Normaldruck über eine Vigreux-Kolonne eingeengt und der Rückstand wurde bei 125 mbar fraktioniert destilliert.

Der 1-Methylcyclopropancarboxaldehyd ging bei 50 bis 52 °C über. Die Ausbeute betrug 27 g (49,2 % der Theorie).

Beispiel 2:

25 g (0,21 Mol) 4-Chlor-2-methyl-butanal wurden bei -25 °C zu einer Suspension von 50 g (0,46 Mol) Lithiumdiisopropylamid in 200 ml absolutem Diethylether langsam innerhalb von 30 Minuten zugetropft. Nach beendeter Zugabe wurde das Reaktionsgemisch auf Raumtemperatur kommen gelassen und danach noch 1 Stunde auf Rückflußtemperatur erwärmt. Anschließend wurde unter Kühlung langsam Wasser zugetropft. Die organische Phase wurde abgetrennt, mit 5-gewichtsprozentiger Salzsäure, dann mit Natriumcarbonatlösung und schließlich mit Wasser ausgewaschen und weiterbehandelt wie im Beispiel 1.

Die Ausbeute an 1-Methylcyclopropancarboxaldehyd betrug 11,8 g (67,6 % der Theorie).

Beispiel 3:

60,3 g (0,5 Mol) 4-Chlor-2-methyl-butanal wurden bei 0 °C zu einer Suspension von 12 g (0,5 Mol) Natriumhydrid in 250 ml Diethylenglykoldimethylether langsam zugetropft. Das Reaktionsgemisch wurde auf Raumtemperatur kommen gelassen und noch 2 Stunden auf 60 °C erwärmt. Anschließend wurde es direkt unter Normaldruck über eine Vigreux-Kolonne fraktioniert destilliert.

Der 1-Methylcyclopropancarboxaldehyd ging bei 103 bis 107 °C über. Die Ausbeute betrug 14,2 g (33,8 % der Theorie).

Beispiel 4:

24,1 g (0,2 Mol) 4-Chlor-2-methyl-butanal wurden bei 0 °C zu einer Suspension von 16 g (0,67 Mol) Natriumhydrid in 150 ml Benzol innerhalb einer Stunde zugetropft. Nach beendeter Zugabe wurde das Reaktionsgemisch auf Raumtemperatur kommen gelassen und noch 2 Stunden auf 80 °C erwärmt. Die Aufarbeitung erfolgte wie im Beispiel 1.

Die Ausbeute an 1-Methylcyclopropancarboxaldehyd betrug 9,2 g (55,0 % der Theorie).

Beispiel 5:

12 g (0,1 Mol) 4-Chlor-2-methyl-butanal wurden bei -10 °C langsam zu 90 g (0,2 Mol) einer 15-gewichtsprozentigen Lösung von tert.Butyllithium in einem Pentan/Hexan-Gemisch zugetropft. Nach beendeter Zugabe wurde das Reaktionsgemisch auf Raumtemperatur kommen gelassen und danach noch 2 Stunden auf Rückflußtemperatur erwärmt. Die Aufarbeitung erfolgte wie im Beispiel 1.

Die Ausbeute an 1-Methylcyclopropancarboxaldehyd betrug 5,2 g (62,0 % der Theorie).

Degussa Aktiengesellschaft
Weissfrauenstrasse 9, 6000 Frankfurt

# Verfahren zur Herstellung von 1-Methylcyclopropancarboxaldehyd

## Patentansprüche:

1. Verfahren zur Herstellung von 1-Methylcyclopropancarboxaldehyd der Formel

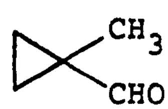

(I),

dadurch gekennzeichnet, daß man 4-Chlor-2-methyl-butanal der Formel

$$ClH_2C - CH_2 - \underset{\displaystyle CH_3}{\underset{|}{CH}} - CHO \qquad (II)$$

bei einer Temperatur zwischen -40 und +70 °C in einem inerten Lösungsmittel mit der 1,0- bis 10,0-fachen molaren Menge an einer starken, nicht nucleophilen Base behandelt.

2. Verfahren nach Anspruch 1, dadurch gekennzeichnet, daß man die Behandlung mit der Base bei einer Temperatur zwischen -20 und +20 °C vornimmt.

3. Verfahren nach Anspruch 1 oder 2, dadurch gekennzeichnet, daß man die Base in der 1,0- bis 4,0-fachen molaren Menge einsetzt.